# EUROPEAN PATENT APPLICATION

(11) **EP 4 768 012 A1**
(43) Date of publication of application: **01.07.2026**
(21) Application number: 24891354.3
(22) Date of filing: 11.11.2024
(51) Int. Cl.: A61K 8/31, A61K 8/34, A61K 8/37, A61K 8/39, A61Q 17/04

(54) **SUNSCREEN COMPOSITION**

(30) Priority: 16.11.2023 JP 2023195221
(71) Applicant: NOF Corporation, Shibuya-ku Tokyo 150-6019 (JP)
(72) Inventor: MUNEKATA Yuki, Amagasaki-shi, Hyogo 660-0095 (JP); SAKATSUME Yuma, Amagasaki-shi, Hyogo 660-0095 (JP); MORIKAWA Toshiyuki, Amagasaki-shi, Hyogo 660-0095 (JP)
(74) Representative: Williams Powell
(86) International application number: PCT/JP2024/039901
(87) International publication number: WO 2025/105318

(57) **Abstract**

A sunscreen composition including, from 2 to 51 mass% of Component (A), from 9 to 80 mass% of Component (B), from 0.1 to 14 mass% of Component (C), from 0.1 to 10 mass% of Component (D), from 5 to 40 mass% of Component (E), and from 6 to 57 mass% of Component (F). In the sunscreen composition, the mass ratio of Component (A) to Component (B) ((A)/(B)) is from 5/95 to 60/40, Component (A) is an isoparaffin having an average carbon number from 50 to 250, Component (B) is an isoparaffin having an average carbon number from 20 to 24, Component (C) is an emulsifier such as a sorbitan fatty acid ester having an HLB of from 2 to 10, Component (D) is polyhydric alcohol having from 2 to 6 carbon atoms and from 2 to 3 hydroxyl groups, Component (E) is an ultraviolet scattering agent, and Component (F) is water.

## Description

### Technical field

The present invention relates to a sunscreen composition.

### Background Art

There is growing demand for sunscreen compositions having not only the function of skin protection from ultraviolet damage, but also an excellent feel in use. Such sunscreen compositions are required to provide a rich texture during spreading, a feeling of thickness that causes a feeling of skin protection, and ease of spreading.

For example, PTL 1 describes a makeup cosmetic preparation having ultraviolet protection properties, which includes a specific dextrin fatty acid ester and a non-volatile phenyl-modified silicone, and thereby can be applied thickly and adhere closely to the skin. PTL 2 describes a sunscreen cosmetic preparation which includes a specific fatty acid alkanolamide derivative and thereby exhibits good extensibility when applied.

On the other hand, in recent years, it has become common knowledge that ultraviolet rays have a negative effect on the skin, and sunscreen cosmetic preparations are frequently used even in winter when the amount of ultraviolet rays is low. When such a sunscreen cosmetic preparation is applied to the skin, an ultraviolet scattering agent and another film-forming substance contained in the sunscreen cosmetic preparation cause a sensation of tightness on the skin, that is, a feeling of tightness. Furthermore, in dry air in winter or the like, the skin becomes dry, and thus a feeling of tightness is more easily perceived. That is, in addition to the above-mentioned problems of feeling of thickness and ease of spreading upon application at low temperatures, problems regarding sunscreen cosmetic preparations further include the problem of feeling of tightness.

Although the makeup cosmetic preparation described in PTL 1 is excellent in terms of feeling of thickness, a sunscreen cosmetic preparation prepared using the makeup cosmetic preparation may be inferior in terms of extensibility at low temperatures and the feeling of tightness. The cosmetic preparation composition described in PTL 2 has excellent extensibility upon application, but may be inferior in the feeling of thickness and feeling of tightness.

That is, in low temperatures such as in winter, these cosmetic preparations may be inferior not only in the effect of being easy to spread while maintaining the feeling of thickness upon application, but also in the feeling of tightness.

To solve the problem of the feeling of tightness, PTL 3 discloses a sunscreen cosmetic preparation containing a specific fine particulate metal oxide, an aqueous phase thickener, and an amphoteric surfactant, and states that this sunscreen cosmetic preparation does not cause a feeling of tightness and provides an excellent feel in use.

### Citation List

### Patent Literature

PTL 1: JP 2017-114832 A
PTL 2: JP 2018-52864 A
PTL 3: JP 2021-138621 A

### Summary of Invention

### Technical Problem

However, in winter sports such as skiing and snowboarding, the skin is exposed to both dryness and wind. Thus, while no feeling of tightness is perceived immediately after applying a sunscreen cosmetic preparation, the effect thereof may be lost with the passage of time. This is because under such circumstances, some components such as a moisturizing component tend to volatilize more easily, causing dryness and stiffness on the skin. The sunscreen cosmetic preparation of PTL 3 may lose the effectiveness over time, and thus there has been a demand for a sunscreen cosmetic preparation that does not cause a feeling of tightness even after activity in a dry or windy environment, and that provides an excellent feel in use in terms of a feeling of thickness and ease of spreading.

In view of the above problems, an object of the present invention is to provide a sunscreen composition that is easy to spread while maintaining a feeling of thickness upon application even in a low-temperature environment, suppresses a feeling of tightness over time even under dry or windy conditions, and thus provides an excellent feel in use.

### Solution to Problem

To solve the above problems, the inventor(s) conducted extensive research and found that, by combining two specific types of isoparaffin, an emulsifier, and a specific polyhydric alcohol, it is possible to produce a sunscreen composition containing an ultraviolet scattering agent, which can solve the above problems of the feeling of thickness, ease of spreading, and the feeling of tightness over time under dry or windy conditions, and thus completed the present invention.

That is, the present invention is a sunscreen composition including, from 2 to 51 mass% of Component (A), from 9 to 80 mass% of Component (B), from 0.1 to 14 mass% of Component (C), from 0.1 to 10 mass% of Component (D), from 5 to 40 mass% of Component (E), and from 6 to 57 mass% of Component (F). In the sunscreen composition, the mass ratio of Component (A) to Component (B) ((A)/(B)) is from 5/95 to 60/40, Component (A) is an isoparaffin having an average carbon number from 50 to 250, Component (B) is an isoparaffin having an average carbon number from 20 to 24, Component (C) is one or more emulsifiers selected from the group consisting of sorbitan fatty acid esters having an HLB of from 2 to 10, glyceryl fatty acid esters having an HLB of from 2 to 10, and polyoxyethylene glyceryl monofatty acid esters having an HLB of from 2 to 10, Component (D) is polyhydric alcohol having from 2 to 6 carbon atoms and from 2 to 3 hydroxyl groups, Component (E) is an ultraviolet scattering agent, and Component (F) is water.

### Advantageous Effects of Invention

According to the sunscreen composition of the present invention, it is possible to achieve the effect of providing ease of spreading while maintaining a feeling of thickness upon application even in a low-temperature environment, and the effect of suppressing a feeling of tightness over time even under dry or windy conditions.

### Description of Embodiments

Hereinafter, embodiments of the present invention will be described. However, the present invention is not limited to the embodiments described herein, and various modifications are possible without departing from the spirit and scope of the present invention.

Note that, as used herein, any numerical range defined using "from ... to" is intended to include numerical values before and after "to" (the upper and lower limits). For example, "from 2 to 5" means 2 or more and 5 or less.

Furthermore, regarding any numerical range described herein, the upper or lower limit value of the numerical range can be replaced with the upper or lower limit value of another numerical range, a value presented in an example, or a value that is uniquely derived from an example.

It is understood that values described herein are subject to the inherent variation characteristic of a measurement technique used to determine the values. In addition, numerical values should be interpreted in light of the number of significant digits and by applying rounding techniques.

### <Sunscreen Composition>

The sunscreen composition of the present invention contains Component (A), Component (B), Component (C), Component (D), Component (E), and Component (F). Each component will be explained below.

### [Component (A)]

Component (A) used in the present invention is an isoparaffin having an average carbon number from 50 to 250. This isoparaffin is a mixture of long-chain hydrocarbons having a side chain, and is typically obtained by hydrogenating a polymer made from isobutene and n-butene. Such components may include those generally classified as "heavy liquid isoparaffins" in the cosmetics field.

The lower limit value of the average carbon number of the isoparaffin is 50, preferably 60, more preferably 70, and particularly preferably 80. The upper limit value of the average carbon number of the isoparaffin is 250, preferably 230, more preferably 210, and particularly preferably 190. If the average carbon number is too small, a feeling of thickness upon application of the sunscreen composition may decrease in a low-temperature environment, and a feeling of tightness over time may be easily perceived. On the other hand, if the average carbon number is too large, the ease of spreading in a low-temperature environment may decrease.

The range of the average carbon number of the isoparaffins can be defined using any values selected from the upper and lower limit values listed above. The average carbon number of the isoparaffin is, for example, from 50 to 250, preferably from 60 to 230, more preferably from 70 to 210, and particularly preferably from 80 to 190.

The average carbon number in this specification is a value derived from peak area measurements and results of mass analysis obtained by using a gas chromatography-mass spectrometry (GC-MS) measuring device (JMS-T2000GC AccuTOF (registered trademark) GC-Alpha, manufactured by JEOL Ltd.).

Component (A) can be synthesized, for example, using the following method. First, a mixed gas of isobutene and n-butene can be processed using a known method, for example, cationic polymerization using a catalyst, to obtain a polymer. Then, the resulting polymer can be hydrogenated to obtain a hydrogenated polymer, which can be subjected to purification such as adsorption treatment and distillation to obtain Component (A).

Specific examples of Component (A) include "PARLEAM 18" (having an average carbon number of 72), "PARLEAM 24" (having an average carbon number of 96), and "PARLEAM 46" (having an average carbon number of 184) (all manufactured by NOF CORPORATION). As Component (A), one or more of these isoparaffins having different average carbon numbers can be used.

### [Component (B)]

Component (B) used in the present invention is an isoparaffin having an average carbon number from 20 to 24, in other words, is a mixture of medium-chain hydrocarbons having a side chain, and is typically obtained by hydrogenating a polymer made from isobutene and n-butene. Such components may include those generally classified as "liquid isoparaffins" in the cosmetics field.

If the average carbon number is less than 20, the feeling of thickness upon application of the sunscreen composition may decrease in a low-temperature environment, and the feeling of tightness over time may be easily perceived. On the other hand, if the average carbon number is more than 24, the ease of spreading in a low-temperature environment may decrease.

Component (B) can be synthesized, for example, using the following method. First, a mixed gas of isobutene and n-butene can be processed using a known method, for example, cationic polymerization using a catalyst, to obtain a polymer. Then, the resulting polymer can be hydrogenated to obtain a hydrogenated polymer, which can be subjected to purification such as adsorption treatment and distillation to obtain Component (B).

Specific examples of Component (B) include "PARLEAM 6" (having an average carbon number of 24) and "PARLEAM EX" (having an average carbon number of 20) (which are manufactured by NOF CORPORATION), and IP SOLVENT 1620 (having an average carbon number of 20) (manufactured by Idemitsu Kosan Co.,Ltd.). As Component (B), one or more of these isoparaffins having different average carbon numbers can be used.

### [Component (C)]

Component (C) used in the present invention is one or more emulsifiers selected from the group consisting of sorbitan fatty acid esters having an HLB of from 2 to 10, glyceryl fatty acid esters having an HLB of from 2 to 10, and polyoxyethylene glyceryl monofatty acid esters having an HLB of from 2 to 10.

The lower limit value of the HLB of the emulsifier of Component (C) is 2, preferably 3, and more preferably 4. The upper limit value of the HLB is 10, preferably 8, and more preferably 6. The range of HLB can be defined using any values selected from the upper and lower limit values listed above. The HLB of Component (C) is, for example, from 2 to 10, preferably from 3 to 8, and more preferably from 4 to 6. If the HLB of Component (C) is outside the range defined above, the emulsion stability may decrease.

The HLB mentioned here is an index of affinity for water, and is calculated by using the following equation according to Griffin (W. C. Griffin: J. Soc. Cosmetic Chemists, 33, 1180 (1960)).
HLB = 20(1 - S/A) (where S is the saponification value of an ester, and A is the neutralization value of a fatty acid)

The saponification value and neutralization value can be measured, for example, according to a method described in "Standard Methods for the Analysis of Fats, Oils and Related Materials (1)" (Japan Oil Chemists' Society, 1996).

The constituent fatty acids of the sorbitan fatty acid ester, glyceryl fatty acid ester, and polyoxyethylene glyceryl monofatty acid ester preferably have from 8 to 22 carbon atoms. The fatty acid may be branched or straight-chain, and may be a fatty acid having one carboxyl group or a fatty acid having two or more carboxyl groups, but is preferably a fatty acid having one carboxyl group. The fatty acid may be a saturated fatty acid or an unsaturated fatty acid, may have a hydroxy group, or may be a condensation product of a fatty acid. The fatty acid may be a fat and oil mixture that is a mixture of fatty acids. Furthermore, in addition to the fatty acids having from 8 to 22 carbon atoms, the fatty acid used in production may include rosin acid in an amount that does not impair the effects of the present invention.

The fatty acid has preferably 12 or more carbon atoms, more preferably 14 or more carbon atoms, and has preferably 18 or less carbon atoms. Preferred ranges of the number of carbon atoms in the fatty acid include, in addition to from 8 to 22, for example, from 12 to 22, from 14 to 22, from 8 to 18, from 12 to 18, and from 14 to 18.

Examples of the fatty acid having from 8 to 22 carbon atoms include ethylhexanoic acid, capric acid, lauric acid, myristic acid, palmitic acid, isopalmitic acid, macadamia nut fatty acids, stearic acid, isostearic acid, hydroxystearic acid, oleic acid, and behenic acid. Among them, myristic acid, palmitic acid, isopalmitic acid, stearic acid, isostearic acid, and oleic acid are preferred.

Sorbitan fatty acid esters are esters having a structure with an ester bond formed by one hydroxy group of sorbitol or a sorbitol intramolecular condensation product and a fatty acid. Examples of the sorbitan fatty acid ester having an HLB of from 2 to 10 include commercially available products such as NONION CP-08R (sorbitan monocaprate (HLB: 9.6)), NONION LP-20R (sorbitan monolaurate (HLB: 8.6)), NONION PP-40R PELLET (sorbitan monopalmitate (HLB: 6.7)), NONION SP-60R PELLET (sorbitan monostearate (HLB: 4.7)), NONION OP-80R (sorbitan monooleate (HLB: 4.3)), and NONION OP-83RAT (sorbitan sesquioleate (HLB: 3.7)) (all manufactured by NOF CORPORATION).

The glyceryl fatty acid ester is an ester having a structure with an ester bond formed by one hydroxy group of glycerin and a fatty acid, and the polyoxyethylene glyceryl monofatty acid ester is a compound having a structure in which ethylene oxide is added to the remaining two hydroxy groups of the ester. The lower limit value of the number of moles of ethylene oxide added in the polyoxyethylene glyceryl monofatty acid ester is preferably 5, more preferably 6, and particularly preferably 7, and the upper limit value thereof is preferably 30, more preferably 20, and particularly preferably 10. The range of the number of moles of ethylene oxide added can be defined using any values selected from the upper and lower limit values listed above. The number of moles of ethylene oxide added in the polyoxyethylene glyceryl monofatty acid ester is, for example, preferably from 5 to 30, more preferably from 6 to 20, and particularly preferably from 7 to 10.

Examples of the glyceryl fatty acid ester include glyceryl myristate and glyceryl coconut oil fatty acid, and examples of the polyoxyethylene glyceryl monofatty acid ester include polyoxyethylene glyceryl coconut oil fatty acid, polyoxyethylene (caprylic/capric) glyceride, polyoxyethylene glyceryl laurate, polyoxyethylene glyceryl oleate, and polyoxyethylene glyceryl isostearate. As the glyceryl fatty acid ester having an HLB of from 2 to 10, for example, commercially available products such as NIKKOL MGM (glyceryl myristate (HLB: 3.5)) (manufactured by NIKKO CHEMICALS) can be used.

As Component (C), one selected from these esters which are emulsifiers may be used alone, or two or more of these esters may be used in combination.

### [Component (D)]

Component (D) used in the present invention is a polyhydric alcohol having from 2 to 6 carbon atoms and from 2 to 3 hydroxyl groups, and one or more selected from the polyhydric alcohols that satisfy these conditions can be used. Among these, polyhydric alcohols having from 2 to 5 carbon atoms are preferred, and polyhydric alcohols having 3 or 4 carbon atoms are more preferred. In addition, trihydric alcohols are preferred. Specific examples include propylene glycol, 1,3-butylene glycol, and glycerin, and 1,3-butylene glycol and glycerin are preferred, and glycerin is more preferred. Dihydric or trihydric means that a single molecule contains two or three hydroxy groups.

### [Component (E)]

Component (E) used in the present invention is an ultraviolet scattering agent included for the purpose of scattering or blocking ultraviolet rays. The ultraviolet scattering agent is not particularly limited as long as the ultraviolet scattering agent is one commonly used in cosmetics, but is preferably at least one selected from the group consisting of titanium oxide, zinc oxide, iron oxide, and cerium oxide. Among these, titanium oxide and zinc oxide are more preferred.

These ultraviolet scattering agents preferably have an average primary particle size of from 5 nm to 100 nm, and more preferably from 10 nm to 40 nm.

Note that the "average primary particle size" is a value obtained by measuring the particle sizes of 200 randomly selected particles using a transmission electron microscope and calculating the average of the primary particle sizes.

The shape of the ultraviolet scattering agent is not particularly limited, and ultraviolet scattering agents having any shape, such as a spherical shape, a rod-like shape, a needle-like shape, a spindle-like shape, or a plate-like shape, can be used.

The ultraviolet scattering agent may be coated with either an inorganic surface coating or an organic surface coating, or may be coated with both an inorganic surface coating and an organic surface coating.

The inorganic surface coating means that the surface of the ultraviolet scattering agent is coated with, for example, a hydroxide and/or oxide of at least one element selected from silicon, aluminum, zinc, iron, titanium, and zirconium, and among these, coating with aluminum hydroxide is preferred.

The organic surface coating means that the surface of the ultraviolet scattering agent is coated with a known organosilicon compound, for example, a known silicone having a hydrogen-silicon bond such as methylhydrogenpolysiloxane (dimethicone/methicone) copolymer, or triethoxysilylethylpolydimethylsiloxyethyldimethicone having an alkoxy group-silicon bond as a reactive group, and among these, coating with methylhydrogenpolysiloxane (dimethicone/methicone) copolymer is more preferred.

These ultraviolet scattering agents may be used in their pure form, or commercially available products in which metal oxide are stably reduced to low-order particles in an oil agent may be used. Examples of oil agents used to form low-order particles of metal oxide include silicone oils, ester oils, and hydrocarbon oils. Examples of such commercially available products include "IOPP60ZIAJ" and "IOPP50TIJ" manufactured by Kobo Dispatek, inc., and "DIF-3ST2", "DIS-11A", and "STR-100A-LP" manufactured by SAKAI CHEMICAL INDUSTRY CO.,LTD.

"IOPP60ZIAJ" manufactured by Kobo Dispatek, inc. includes zinc oxide, ethylhexyl palmitate, isostearic acid, and polyhydroxystearic acid, and "IOPP50TIJ" includes titanium oxide, ethylhexyl palmitate, aluminum hydroxide, isostearic acid, and polyhydroxystearic acid. "DIF-3ST2" manufactured by SAKAI CHEMICAL INDUSTRY CO.,LTD. includes zinc oxide, cyclopentasiloxane, and hydrogen dimethicone, "DIS-11A" includes titanium oxide, cyclopentasiloxane, hydrated silica, aluminum hydroxide, and hydrogen dimethicone, and "STR-100A-LP" includes titanium oxide, hydrated silica, aluminum hydroxide, and hydrogen dimethicone.

One of the ultraviolet scattering agents can be used alone or two or more thereof can be used in combination.

### [Component (F)]

Component (F) used in the present invention is water, examples thereof include purified water such as ion-exchanged water and distilled water, and tap water, and purified water such as ion-exchanged water and distilled water is preferred.

### [Content of Each Component]

The lower limit value of the content of Component (A) in the sunscreen composition is 2 mass%, preferably 5 mass%, more preferably 10 mass%, and particularly preferably 14 mass%. The upper limit value of the content of Component (A) is 51 mass%, preferably 35 mass%, more preferably 25 mass%, and particularly preferably 18 mass%. If the content of Component (A) is too low, the feeling of tightness over time may be easily perceived and the feeling of thickness upon application may decrease in a low-temperature environment. On the other hand, if the content of Component (A) is too high, the ease of spreading in a low-temperature environment may decrease.

The range of the content of Component (A) can be defined using any values selected from the upper and lower limit values listed above. The content of Component (A) is, for example, from 2 to 51 mass%, preferably from 5 to 35 mass%, more preferably from 10 to 25 mass%, and particularly preferably from 14 to 18 mass%.

The lower limit value of the content of Component (B) in the sunscreen composition is 9 mass%, preferably 17 mass%, more preferably 25 mass%, and particularly preferably 33 mass%. The upper limit value of the content of Component (B) is 80 mass%, preferably 66 mass%, more preferably 52 mass%, and particularly preferably 39 mass%. If the content of Component (B) is too low, the ease of spreading in a low-temperature environment may decrease, and the feeling of tightness over time may be easily perceived. On the other hand, if the content of Component (B) is too high, the feeling of thickness upon application may decrease in a low-temperature environment.

The range of the content of Component (B) can be defined using any values selected from the upper and lower limit values listed above. The content of Component (B) is, for example, from 9 to 80 mass%, preferably from 17 to 66 mass%, more preferably from 25 to 52 mass%, and particularly preferably from 33 to 39 mass%.

The lower limit value of the content of Component (C) in the sunscreen composition is 0.1 mass%, preferably 1 mass%, more preferably 2 mass%, and particularly preferably 3 mass%. The upper limit value of the content of Component (C) is 14 mass%, preferably 10 mass%, more preferably 8 mass%, and particularly preferably 6 mass%. If the content of Component (C) is too low, the emulsion stability may become insufficient, whereas if the content of Component (C) is too high, the ease of spreading in a low-temperature environment may decrease.

The range of the content of Component (C) can be defined using any values selected from the upper and lower limit values listed above. The content of Component (C) is, for example, from 0.1 to 14 mass%, preferably from 1 to 10 mass%, more preferably from 2 to 8 mass%, and particularly preferably from 3 to 6 mass%.

The lower limit value of the content of Component (D) in the sunscreen composition is 0.1 mass%, preferably 1 mass%, more preferably 2 mass%, and particularly preferably 3 mass%. The upper limit value of the content of Component (D) is 10 mass%, preferably 8 mass%, more preferably 7 mass%, and particularly preferably 6 mass%. If the content of Component (D) is too low, the feeling of thickness upon application may decrease in a low-temperature environment. On the other hand, if the content of Component (D) is too high, the ease of spreading in a low-temperature environment may decrease.

The range of the content of Component (D) can be defined using any values selected from the upper and lower limit values listed above. The content of Component (D) is, for example, from 0.1 to 10 mass%, preferably from 1 to 8 mass%, more preferably from 2 to 8 mass%, particularly preferably from 3 to 7 mass%, and even more preferably from 3 to 6 mass%.

The lower limit value of the content of Component (E) in the sunscreen composition is 5 mass%, preferably 10 mass%, more preferably 15 mass%, and particularly preferably 20 mass%. The upper limit value of the content of Component (E) is 40 mass%, preferably 35 mass%, more preferably 30 mass%, and particularly preferably 25 mass%. If the content of Component (E) is too low, a sufficient ultraviolet protection effect may not be achieved, whereas if the content of Component (E) is too high, the ease of spreading in a low-temperature environment may decrease and the feeling of tightness over time may be easily perceived.

The range of the content of Component (E) can be defined using any values selected from the upper and lower limit values listed above. The content of Component (E) is, for example, from 5 to 40 mass%, preferably from 10 to 35 mass%, more preferably from 15 to 30 mass%, particularly preferably from 20 to 30 mass%, and even more preferably from 20 to 25 mass%.

The lower limit value of the content of Component (F) in the sunscreen composition is 6 mass%, preferably 10 mass%, more preferably 16 mass%, and particularly preferably 18 mass%. The upper limit value of the content of Component (F) is 57 mass%, preferably 47 mass%, more preferably 35 mass%, and particularly preferably 25 mass%. If the content of Component (F) is too low, the feeling of tightness over time may be easily perceived, whereas if the content of Component (F) is too high, the emulsion stability may become insufficient, the feeling of tightness over time may be easily perceived, and the feeling of thickness upon application may decrease in a low-temperature environment.

The range of the content of Component (F) can be defined using any values selected from the upper and lower limit values listed above. The content of Component (F) is, for example, from 6 to 57 mass%, preferably from 10 to 47 mass%, more preferably from 16 to 47 mass%, particularly preferably from 18 to 35 mass%, and even more preferably from 18 to 25 mass%.

In the present invention, the lower limit value of the mass ratio of Component (A) to Component (B) ((A)/(B)) is 5/95, preferably 10/90, and more preferably 20/80. The upper limit value of the mass ratio ((A)/(B)) is 60/40, preferably 50/50, and more preferably 40/60. If the mass ratio ((A)/(B)) is too small, the feeling of thickness upon application may decrease in a low-temperature environment, and the feeling of tightness over time may be easily perceived. If the mass ratio ((A)/(B)) is too large, the ease of spreading in a low-temperature environment may decrease.

The range of the mass ratio ((A)/(B)) can be defined using any mass ratios selected from the upper and lower limit values listed above. The mass ratio ((A)/(B)) is, for example, from 5/95 to 60/40, preferably from 10/90 to 50/50, and more preferably from 20/80 to 40/60.

### [Other Components]

Various other components may be added to the sunscreen composition of the present invention as long as the stability of the composition can be maintained and the effects of the present invention are not impaired. Examples of the other components include oily materials such as vegetable oils and fats, animal oils and fats, wax, paraffins other than polybutene, petrolatum, fatty acid esters other than Component (C), higher fatty acids, and higher alcohols; inorganic compounds such as talc, silica, kaolin, sodium carbonate, and borax; organic solvents such as ethanol, isopropanol, and ethylene glycol; water-soluble polymers such as polyethylene glycol, polypropylene glycol, polyvinyl alcohol, polyvinylpyrrolidone, carboxymethyl cellulose, block copolymers of ethylene oxide and propylene oxide, and copolymers of maleic anhydride and methyl vinyl ether; and other additives such as oxidation stabilizers, preservatives, disinfectants, colorants, fragrances, pharmaceuticals, and mixtures thereof.

The sunscreen composition of the present invention may contain the above-mentioned compound(s) other than an ultraviolet scattering agent contained in a commercially available ultraviolet scattering agent of Component (E).

The content of the above-mentioned other component(s) in the sunscreen composition of the present invention may be set according to the amount typically used in cosmetics and the like depending on the purpose, but is typically 20 mass% or less in the composition. The upper limit value of the content of the other component(s) is preferably 15 mass% and more preferably 10 mass%, and the lower limit value thereof is preferably 0.1 mass% and more preferably 0.5 mass%.

A preferred range of the content of other component(s) can be defined using any values selected from the upper and lower limit values listed above. The content of the other component(s) is, for example, preferably from 0.1 to 15 mass% and more preferably from 0.5 to 10 mass%.

The content of each of the above components is expressed as the content when the total content of all components of the sunscreen composition is 100 mass%. The total content of Components (A) to (F) described above is preferably 80 mass% or more, more preferably 85 mass% or more, and particularly preferably 90 mass% or more. Note that, by appropriately adjusting the amount of water added, the content of each component can be adjusted to the desired level.

### [Production of Sunscreen Composition]

The sunscreen composition of the present invention can be produced by known methods. For example, the sunscreen composition can be produced by mixing the above-mentioned components and emulsifying the mixture using an emulsifying machine or kneader such as a homogenizer, homogenizing mixer, roll mill, or mill, in a temperature range of from room temperature to 90°C for about 10 to 120 minutes depending on the volume.

### Examples

Hereinafter, embodiments of the present invention will be more specifically described with reference to examples and comparative examples, but the present invention is not limited to these examples.

### <Sunscreen Composition>

Sunscreen compositions shown in Table 1 (Examples 1 to 16) and Table 2 (Comparative Examples 1 to 10) were prepared using a known method and evaluated using the following methods.

Note that, in Tables 1 and 2, the numerical value of each component is the content value (mass%) relative to the total amount of the sunscreen composition. Component (A)/Component (B) shows the content ratio of Component (A) to Component (B).

### [Component (A)]

#1 Hydrogenated polyisobutene: PARLEAM 24 (NOF CORPORATION) (average carbon number, 96)
#2 Hydrogenated polyisobutene: PARLEAM 46 (NOF CORPORATION) (average carbon number, 184)
#3 Hydrogenated polyisobutene: PARLEAM 18 (NOF CORPORATION) (average carbon number, 72)

### [Component (B)]

#4 Hydrogenated polyisobutene: PARLEAM 6 (NOF CORPORATION) (average carbon number, 24)
#5 Hydrogenated polyisobutene: PARLEAM EX (NOF CORPORATION) (average carbon number, 20)

### [Component (C)]

#6 Sorbitan monooleate: NONION OP-80R (NOF CORPORATION, HLB: 4.3)
#7 Glyceryl myristate: NIKKOL MGM (NIKKO CHEMICALS, HLB: 3.5)

### [Component (D)]

- Glycerin: RG-S (NOF CORPORATION)
- 1,3-butylene glycol: 1,3-butylene glycol-P (KH Neochem Co., Ltd.)

### [Component (E)]

#8 Ultraviolet scattering agent (titanium oxide): STR-100A-LP (SAKAI CHEMICAL INDUSTRY CO.,LTD.) (average primary particle size, 15 nm)

### <Evaluation of Sunscreen Compositions>

Each of the sunscreen compositions of Examples 1 to 16 and Comparative Examples 1 to 10 was evaluated for a feeling of thickness upon application, ease of spreading at low temperatures, and suppression of a feeling of tightness over time, as described in (1) to (3) below. The evaluation results are shown in Tables 1 and 2.

Note that panelists conducting the following evaluations were trained in a preliminary evaluation test conducted prior to the evaluation tests such that the levels of evaluation corresponding to their respective scores were roughly the same, to equalize evaluation criteria of the panelists.

### (1) Feeling of Thickness upon Application

Ten male and female panelists, aged between 25 and 55, washed their hands with hand soap, then took 0.2 g of each sunscreen composition that had been stored at 5°C onto their fingertips and applied it to the back of their hands. At that time, the panelists evaluated a rich texture during spreading and a feeling of skin protection, using the absolute evaluation criteria below.

### <Absolute Evaluation Criteria>

### (Score): (Evaluation)

2 points: A significant feeling of thickness was perceived.
1 point: A slight feeling of thickness was perceived.
0 points: No feeling of thickness was perceived.

### (2) Ease of Spreading at Low Temperatures

Ten male and female panelists, aged between 25 and 55, washed their hands with hand soap, then took 0.2 g of each sunscreen composition that had been stored at 5°C onto their fingertips and applied it to the back of their hands. The panelists evaluated extensibility of the composition upon the application, using the absolute evaluation criteria below.

### <Absolute Evaluation Criteria>

### (Score): (Evaluation)

2 points: Excellent extensibility was perceived.
1 point: Slightly good extensibility was perceived.
0 points: Poor extensibility was perceived.

### (3) Suppression of Feeling of Tightness over Time

Ten male and female panelists, aged between 25 and 55, took 0.2 g of each sunscreen composition that had been stored at 5°C onto their fingertips and applied it to the back of their hands. Thereafter, the panelists spent three hours in an environment at 10°C and 30% humidity. Then, the panelists evaluated moist feeling of the skin similar to that immediately after the application, and absence of a sensation of skin tightness when moving the fingers. During the three hours, air was blown onto the back of the hand with a fan once every 30 minutes for three minutes each time. The evaluation was carried out according to the following absolute evaluation criteria.

### <Absolute Evaluation Criteria>

### (Score): (Evaluation)

2 points: No feeling of tightness was perceived.
1 point: A slight feeling of tightness was perceived.
0 points: A significant feeling of tightness was perceived.

For each of the evaluations (1) to (3) above, the total of scores of the panelists was calculated, and the total score was evaluated using the following evaluation criteria. The evaluations other than "poor", that is, "excellent (exc.)", "good" and "marginal (marg.)" were considered to be acceptable.
Excellent: Total score of 17 or more
Good: Total score of 14 or more and less than 17
Marginal: Total score of 11 or more and less than 14
Poor: Total score of less than 11

### [Table 1]

**Table 1**

| | | | Exam ples | | | | | | | | | | | | | | | |
|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | 15 | 16 |
| Sunscreen composition | Component (A) | Hydrogenated polyisobutene #1 with average carbon number of 96 | 16 | 8 | 6 | 23 | 43 | 9 | 16 | 17 | 16 | - | - | 16 | 17 | 13 | 16 | 12 |
| | | Hydrogenated polyisobutene #2 with average carbon number of 184 | - | - | - | - | - | - | - | - | - | 16 | - | - | - | - | - | - |
| | | Hydrogenated polyisobutene #3 with average carbon number of 72 | - | - | - | - | - | - | - | - | - | - | 16 | - | - | - | - | - |
| | Component (B) | Hydrogenated polyisobutene #4 with average carbon number of 24 | 36 | 44 | 68 | 29 | 35 | 22 | 36 | 39 | 36 | 36 | 36 | - | 38.5 | 31 | 36 | 27 |
| | | Hydrogenated polyisobutene #5 with average carbon number of 20 | - | - | - | - | - | - | - | - | - | - | - | 36 | - | - | - | - |
| | Component (C) | Sorbitan monooleate #6 | 4 | 4 | 4 | 4 | 4 | 4 | 4 | 4 | 4 | 4 | 4 | 4 | 0.5 | 12 | - | 4 |
| | | Glyceryl myristate #7 | - | - | - | - | - | - | - | - | - | - | - | - | - | - | 4 | - |
| | Component (D) | Glycerin | 4 | 4 | 4 | 4 | 4 | 4 | 1 | 9 | - | 4 | 4 | 4 | 4 | 4 | 4 | 4 |
| | | 1.3-Butylene glycol | - | - | - | - | - | - | - | - | 4 | - | - | - | - | - | - | - |
| | Component (E) | Ultraviolet scattering agent #8 | 21 | 21 | 6 | 21 | 6 | 21 | 21 | 21 | 21 | 21 | 21 | 21 | 21 | 21 | 21 | 36 |
| | Component (F) | Ion-exchanged water | 19 | 19 | 12 | 19 | 8 | 40 | 22 | 10 | 19 | 19 | 19 | 19 | 19 | 19 | 19 | 17 |
| | Component (A)/Component (B) | | 31/69 | 15/85 | 8/92 | 44/56 | 55/45 | 29/71 | 31/69 | 30/70 | 31/69 | 31/69 | 31/69 | 31/69 | 31/69 | 30/70 | 31/69 | 31/69 |
| | Total amount of Components (A) to (F) | | 100 | 100 | 100 | 100 | 100 | 100 | 100 | 100 | 100 | 100 | 100 | 100 | 100 | 100 | 100 | 100 |
| Evaluation | Feeling of thickness upon application (lower row: total score) | | Exc. | Good | Marg. | Exc. | Exc. | Marg. | Marg. | Exc. | Exc. | Exc. | Good | Exc. | Exc. | Exc. | Exc. | Exc. |
| | | | 19 | 15 | 11 | 18 | 20 | 11 | 11 | 18 | 18 | 20 | 15 | 17 | 17 | 17 | 18 | 18 |
| | Ease of spreading at low temperatures (lower row: total score) | | Exc. | Exc. | Exc. | Good | Marg. | Exc. | Exc. | Good | Exc. | Good | Exc. | Exc. | Exc. | Good | Exc. | Good |
| | | | 17 | 17 | 19 | 15 | 11 | 19 | 18 | 15 | 18 | 15 | 17 | 19 | 18 | 16 | 18 | 14 |
| | Suppression of feeling of tightness over time (lower row: total score) | | Exc. | Good | Good | Exc. | Exc. | Good | Good | Exc. | Exc. | Exc. | Good | Exc. | Exc. | Exc. | Exc. | Good |
| | | | 19 | 16 | 14 | 18 | 19 | 14 | 14 | 16 | 18 | 19 | 16 | 17 | 17 | 17 | 18 | 14 |

**Table 2**

| | | | Comparative Examples | | | | | | | | | |
|---|---|---|---|---|---|---|---|---|---|---|---|---|
| | | | 1 | 2 | 3 | 4 | 5 | 6 | 7 | 8 | 9 | 10 |
| Sunscreen composition | Component (A) | Hydrogenated polyisobutene #1 with average carbon number of 96 | 49 | - | 7 | 16 | 4 | 10 | 20 | 3 | 53 | 3 |
| | | Hydrogenated polyisobutene #2 with average carbon number of 184 | - | - | - | - | - | - | - | - | - | - |
| | | Hydrogenated polyisobutene #3 with average carbon number of 72 | - | - | - | - | - | - | - | - | - | - |
| | Component (B) | Hydrogenated polyisobutene #4 with average carbon number of 24 | - | 50 | 35 | 36 | 10 | 23 | 46 | 8 | 28 | 82 |
| | | Hydrogenated polyisobutene #5 with average carbon number of 20 | - | - | - | - | - | - | - | - | - | - |
| | Component (C) | Sorbitan monooleate #6 | 4 | 4 | 15 | 4 | 4 | 4 | 4 | 4 | 4 | 4 |
| | | Glyceryl myristate #7 | - | - | - | - | - | - | - | - | - | - |
| | Component (D) | Glycerin | 4 | 4 | 4 | | 25 | 4 | 4 | 4 | 4 | 4 |
| | | 1,3-Butylene glycol | - | - | - | - | - | - | - | - | - | - |
| | Component (E) | Ultraviolet scattering agent #8 | 21 | 21 | 21 | 21 | 21 | 45 | 21 | 21 | 6 | 6 |
| | Component (F) | Ion-exchanged water | 22 | 21 | 18 | 23 | 36 | 14 | 5 | 60 | 5 | 1 |
| | Component (A)/Component (B) | | 100/0 | 0/100 | 17/83 | 31/69 | 29/71 | 30/70 | 30/70 | 27/73 | 65/35 | 4/96 |
| | Total amount of Components (A) to (F) | | 100 | 100 | 100 | 100 | 100 | 100 | 100 | 100 | 100 | 100 |
| Evaluation | Feeling of thickness upon application (lower row: total score) | | Marg. | Poor | Marg. | Poor | Marg. | Marg. | Exc. | Poor | Marg. | Poor |
| | | | 11 | 1 | 11 | 4 | 11 | 11 | 20 | 4 | 11 | 3 |
| | Ease of spreading at low temperatures (lower row: total score) | | Poor | Exc. | Poor | Exc. | Poor | Poor | Marg. | Exc. | Poor | Exc. |
| | | | 1 | 19 | 3 | 17 | 4 | 0 | 12 | 20 | 1 | 20 |
| | Suppression of feeling of tightness over time (lower row: total score) | | Poor | Poor | Good | Marg. | Marg. | Poor | Poor | Poor | Exc. | Poor |
| | | | 4 | 2 | 14 | 11 | 11 | 1 | 5 | 2 | 17 | 2 |

### [Table 2]

The sunscreen compositions of Examples 1 to 16 all were favorable in the feeling of thickness upon application, ease of spreading at low temperatures, and suppression of the feeling of tightness over time.

In contrast, Comparative Examples 1 to 10 failed to provide sufficient performance.

Comparative Example 1 did not contain Component (B), and thus was insufficient in terms of the ease of spreading at low temperatures and suppression of a feeling of tightness over time.

Comparative Example 2 did not contain Component (A), and thus was insufficient in terms of the feeling of thickness upon application and suppression of a feeling of tightness over time.

Comparative Example 3 contained Component (C) in an amount of more than 14 mass%, and thus was insufficient in terms of the ease of spreading at low temperatures.

Comparative Example 4 did not contain Component (D), and thus was insufficient in terms of the feeling of thickness upon application.

Comparative Example 5 contained Component (D) in an amount of more than 10 mass%, and thus was insufficient in terms of the ease of spreading at low temperatures.

Comparative Example 6 contained Component (E) in an amount of more than 40 mass%, and thus was insufficient in terms of the ease of spreading at low temperatures and suppression of a feeling of tightness over time.

Comparative Example 7 contained Component (F) in an amount of less than 6 mass%, and thus was insufficient in terms of the suppression of a feeling of tightness over time.

Comparative Example 8 contained Component (F) in an amount of more than 57 mass%, and thus was insufficient in terms of the feeling of thickness upon application and suppression of a feeling of tightness over time.

Comparative Example 9 had a mass ratio of Component (A) to Component (B) ((A)/(B)) that is greater than 60/40, and thus was insufficient in terms of the ease of spreading at low temperatures.

Comparative Example 10 had a mass ratio of Component (A) to Component (B) ((A)/(B)) that is less than 5/95, and thus was insufficient in terms of the feeling of thickness upon application and suppression of a feeling of tightness over time.

Next, formulation examples of the sunscreen composition of the present invention (Formulation Examples 1 and 2) are shown. The sunscreen compositions of these formulation examples were also evaluated in terms of the above (1) to (3). As a result, these sunscreen compositions were found to be easy to spread while maintaining the feeling of thickness upon application even in a low-temperature environment, and furthermore, to suppress the feeling of tightness over time even under dry or windy conditions, and thus had the effect of providing an excellent feel in use.

In the following formulation examples, the mass ratio ((A)/(B)) is 34/66 for Formulation Example 1 and 36/64 for Formulation Example 2.

The following Component (E) was used in the following formulation examples.
- Hydrophobized titanium oxide: STR-100A-LP (SAKAI CHEMICAL INDUSTRY CO.,LTD.)
- Hydrophobized zinc oxide: FINEX-50LP (SAKAI CHEMICAL INDUSTRY CO.,LTD.)

### [Formulation Example 1: Sunscreen Milk]

| (Components) | (mass%) |
|---|---|
| 1. Isoparaffin with an average carbon number of 24 (Component (B)) | 31.0 |
| 2. Hydrophobized titanium oxide (Component (E)) | 21.0 |
| 3. Isoparaffin with an average carbon number of 96 (Component (A)) | 16.0 |
| 4. Glycerin (Component (D)) | 5.0 |
| 5. Sorbitan monooleate (Component (C)) | 5.0 |
| 6. (Vinylpyrrolidone/hexadecene) copolymer | 2.5 |
| 7. Stearic acid | 2.0 |
| 8. Preservatives | Proper amount |
| 9. Fragrances | Proper amount |
| 10. Purified water (Component (F)) | 17.0 |
| Total | 100.0 |

### [Formulation Example 2: Sunscreen Milk]

| (Components) | (mass%) |
|---|---|
| 1. Isoparaffin with an average carbon number of 20 (Component (B)) | 30.0 |
| 2. Isoparaffin with an average carbon number of 72 (Component (A)) | 17.0 |
| 3. Hydrophobized titanium oxide (Component (E)) | 11.0 |
| 4. Hydrophobized zinc oxide (Component (E)) | 10.0 |
| 5. Glycerin (Component (D)) | 2.8 |
| 6. 1,3-Butylene glycol (Component (D)) | 2.5 |
| 7. PEG-32 | 2.5 |
| 8. Sorbitan monooleate (Component (C)) | 4.5 |
| 9. Glyceryl myristate (Component (C)) | 0.5 |
| 10. Acrylates/C10-30 alkyl acrylate crosspolymer | 0.5 |
| 11. Citric acid | 0.2 |
| 12. Preservatives | Proper amount |
| 13. Fragrances | Proper amount |
| 14. Purified water (Component (F)) | 18.0 |
| Total | 100.0 |

### Industrial Applicability

The sunscreen composition of the present invention is easy to spread while maintaining a feeling of thickness upon application even in a low-temperature environment, suppresses a feeling of tightness over time even under dry or windy conditions, and provides an excellent feel in use. Thus, the sunscreen composition can be suitably used as a sunscreen cosmetic preparation or the like.

## Claims

1. A sunscreen composition comprising:
from 2 to 51 mass% of Component (A);
from 9 to 80 mass% of Component (B);
from 0.1 to 14 mass% of Component (C);
from 0.1 to 10 mass% of Component (D);
from 5 to 40 mass% of Component (E); and
from 6 to 57 mass% of Component (F),
wherein the mass ratio of Component (A) to Component (B) ((A)/(B)) is from 5/95 to 60/40,
Component (A) is an isoparaffin having an average carbon number from 50 to 250,
Component (B) is an isoparaffin having an average carbon number from 20 to 24,
Component (C) is one or more emulsifiers selected from the group consisting of sorbitan fatty acid esters having an HLB of from 2 to 10, glyceryl fatty acid esters having an HLB of from 2 to 10, and polyoxyethylene glyceryl monofatty acid esters having an HLB of from 2 to 10,
Component (D) is polyhydric alcohol having from 2 to 6 carbon atoms and from 2 to 3 hydroxyl groups,
Component (E) is an ultraviolet scattering agent, and
Component (F) is water.
